# EUROPEAN PATENT APPLICATION

(11) **EP 4 258 253 A1**
(43) Date of publication of application: **11.10.2023**
(21) Application number: 21902191.2
(22) Date of filing: 08.10.2021
(51) Int. Cl.: G09G 5/02, A61B 3/02, G06F 3/14

(54) **METHOD AND APPARATUS FOR ADJUSTING DISPLAY COLOR, ELECTRONIC DEVICE, AND STORAGE MEDIUM**

(30) Priority: 09.12.2020 CN 202011450059
(71) Applicant: GUANGDONG OPPO MOBILE TELECOMMUNICATIONS CORP., LTD., Dongguan, Guangdong 523860 (CN)
(72) Inventor: PENG, Ying, Dongguan, Guangdong 523860 (CN)
(74) Representative: Grassi, Stefano
(86) International application number: PCT/CN2021/122644
(87) International publication number: WO 2022/121483

(57) **Abstract**

Disclosed in the present application are a method and apparatus for adjusting a display color, an electronic device, and a storage medium. The method comprises: in response to a selection operation acting on color blocks, filling the color blocks to controls to be filled; and upon detecting that the color blocks are all filled to the controls to be filed, adjusting the display color on the basis of the relationship between the arrangement order and a preset arrangement order. By adjusting the display color of the electronic device according to a test result, personalization of the display color is achieved, and the user experience is improved.

## Description

### CROSS-REFERENCE OF RELATED APPLICATION

This application claims priority to a Chinese patent application No. CN202011450059.2, filed on December 09, 2020, and the entire contents of which are hereby incorporated by reference.

### TECHNICAL FIELD

The disclosure relates to the technical field of electronic devices, and more particularly to a method and an apparatus for adjusting display colors, an electronic device, and a storage medium.

### BACKGROUND

With the rapid development of image display technology, more and more electronic devices are starting to improve the display effect of colors to enhance the user experience. However, due to varying degrees of recognitions of specific colors by different users, their sensory perception of the same color displayed on an electronic device varies.

### SUMMARY

In view of the above problem, the present disclosure proposes a method for adjusting display colors, an apparatus for adjusting display colors, an electronic device, and a storage medium to solve the above problem.

In a first aspect, an embodiment of the present disclosure provides a method for adjusting display colors, which is implemented by an electronic device. The method includes the following steps. A color testing interface is displayed, the color testing interface includes a first region and a second region, the first region displays a target number of color blocks, the second region displays a target number of controls to be filled, and each of the target number of color blocks is configured to fill one of the target number of controls to be filled. In response to a selection operation acting on one of the target number of color blocks, the one of target number of color blocks is filled to one of the controls to be filled. In response to detecting that the target number of color blocks are all filled to the target number of controls to be filled, display colors of the electronic device are adjusted based on a relationship between arrangement orders of the target number of color blocks on the target number of controls to be filled and preset arrangement orders. Colors of adjacent color blocks of the target number of color blocks with the preset arrangement orders are most similar.

In a second aspect, an embodiment of the present disclosure provides an apparatus for adjusting display colors, which is applied to an electronic device. The apparatus includes a color testing interface display module, a color block moving module, and a display color adjustment module. The color testing interface module is configured to display a color testing interface, the color testing interface includes a first region and a second region, the first region displays a target number of color blocks, the second region displays a target number of controls to be filled, and each of the target number of color blocks is configured to fill one of the target number of controls to be filled. The color block moving module is configured to fill, in response to a selection operation acting on one of the target number of color blocks, the one of target number of color blocks to one of the controls to be filled. The display color adjustment module is configured to in response to detecting that the target number of color blocks are all filled to the target number of controls to be filled, adjust, based on a relationship between arrangement orders of the target number of color blocks on the target number of controls to be filled and preset arrangement orders, display colors of the electronic device. Colors of adjacent color blocks of the target number of color blocks with the preset arrangement orders are most similar.

In a third aspect, an embodiment of the present disclosure provides an electronic device including a memory and a processor, the memory is coupled to the processor, the memory is stored with instructions, and the processor is configured to, when executing the instructions, implement the above method.

In a fourth aspect, an embodiment of the present disclosure provides a computer readable storage medium, in which program codes are stored, and the program codes are configured to, when being called by a processor, implement the above method.

### BRIEF DESCRIPTION OF DRAWINGS

In order to describe technical solutions in embodiments of the present disclosure clearer, a brief introduction will be given to the accompanying drawings required in the description of the embodiments. Apparently, the accompanying drawings in the following description are only some of the embodiments of the present disclosure. For those skilled in the art, other accompanying drawings can be obtained based on these drawings without any creative effort.
FIG. 1 illustrates a schematic flowchart of a method for adjusting display colors according to an embodiment of the present disclosure.
FIG. 2 illustrates a schematic diagram of a first kind of interface of an electronic device according to an embodiment of the present disclosure.
FIG. 3 illustrates a schematic diagram of a second kind of interface of the electronic device according to an embodiment of the present disclosure.
FIG. 4 illustrates a schematic flowchart of a method for adjusting display colors according to another embodiment of the present disclosure.
FIG. 5 illustrates a schematic diagram of a third kind of interface of the electronic device according to an embodiment of the present disclosure.
FIG. 6 illustrates a schematic flowchart of a method for adjusting display colors according to still another embodiment of the present disclosure.
FIG. 7 illustrates a schematic flowchart of a block S330 of the method for adjusting display colors illustrated in FIG. 6 of the present disclosure.
FIG. 8 illustrates a schematic diagram of a fourth kind of interface of the electronic device according to an embodiment of the present disclosure.
FIG. 9 illustrates a schematic diagram of a fifth kind of interface of the electronic device according to an embodiment of the present disclosure.
FIG. 10 illustrates a schematic flowchart of the block S330 of the method for adjusting display colors illustrated in FIG. 6 of the present disclosure.
FIG. 11 illustrates a schematic flowchart of a method for adjusting display colors according to even still another embodiment of the present disclosure.
FIG. 12 illustrates a schematic diagram of a sixth kind of interface of the electronic device according to an embodiment of the present disclosure.
FIG. 13 illustrates a schematic diagram of a seventh kind of interface of the electronic device according to an embodiment of the present disclosure.
FIG. 14 illustrates a schematic flowchart of a method for adjusting display colors according to further still another embodiment of the present disclosure.
FIG. 15 illustrates a schematic diagram of an eighth kind of interface of the electronic device according to an embodiment of the present disclosure.
FIG. 16 illustrates a schematic diagram of a ninth kind of interface of the electronic device according to an embodiment of the present disclosure.
FIG. 17 illustrates a schematic diagram of a tenth kind of interface of the electronic device according to an embodiment of the present disclosure.
FIG. 18 illustrates a schematic diagram of an eleventh kind of interface of the electronic device according to an embodiment of the present disclosure.
FIG. 19 illustrates a schematic diagram of a twelfth kind of interface of the electronic device according to an embodiment of the present disclosure.
FIG. 20 illustrates a schematic diagram of a thirteenth kind of interface of the electronic device according to an embodiment of the present disclosure.
FIG. 21 illustrates a schematic diagram of a fourteenth kind of interface of the electronic device according to an embodiment of the present disclosure.
FIG. 22 illustrates a schematic diagram of a fifteenth kind of interface of the electronic device according to an embodiment of the present disclosure.
FIG. 23 illustrates a schematic diagram of a sixteenth kind of interface of the electronic device according to an embodiment of the present disclosure.
FIG. 24 illustrates a schematic diagram of a seventeenth kind of interface of the electronic device according to an embodiment of the present disclosure.
FIG. 25 illustrates a schematic diagram of an eighteenth kind of interface of the electronic device according to an embodiment of the present disclosure.
FIG. 26 illustrates a schematic block diagram of an apparatus for adjusting display colors according to an embodiment of the present disclosure.
FIG. 27 illustrates a schematic block diagram of the electronic device for implementing the method for adjusting display colors according to an embodiment of the present disclosure.
FIG. 28 illustrates a storage unit for storing or carrying program codes for implementing the method for adjusting display colors according to an embodiment of the present disclosure.

### DETAILED DESCRIPTION OF EMBODIMENTS

In order to enable those skilled in related art to better understand technical solutions of the present disclosure, the technical solutions of embodiments of the present disclosure will be clearly and completely described below with reference to the accompanying drawings of the embodiments of the present disclosure.

Color vision is an important component of human vision, referring to the ability to distinguish and identify colors. Since color perception and response is a complex system full of endless mysteries, the human eye's ability to discriminate colors will be impaired if any part of the process of color discrimination goes wrong. For example, if color vision is weak, it is called color weakness, which usually includes red weakness, green weakness and so on. Because different users have different recognition degrees of specific colors, different users have different senses for the same color displayed on an electronic device. At present, the electronic device uses a unified display color for all users, which is not personalized, resulting in poor user experience.

In view of the above problems, the inventor has discovered after a long period of research and proposed a method for adjusting display colors, an apparatus for adjusting display colors, an electronic device, and a storage medium according to the embodiments of the present disclosure. By providing a color testing interface for sorting similar colors and then adjusting the display colors of the electronic device based on the test results, the display colors are personalized to enhance the user experience. The specific method for adjusting display colors will be described in detail in subsequent embodiments.

In an embodiment of the present disclosure, a method for adjusting display colors is provided and implemented by an electronic device. The method includes the following steps. A color testing interface is displayed, the color testing interface includes a first region and a second region, the first region displays a target number of color blocks, the second region displays a target number of controls to be filled, and each of the target number of color blocks is configured to fill one of the target number of controls to be filled. In response to a selection operation acting on one of the target number of color blocks, the one of the target number of color blocks is filled to one of the controls to be filled. In response to detecting that the target number of color blocks are all filled to the target number of controls to be filled, display colors of the electronic device are adjusted based on a relationship between arrangement orders of the target number of color blocks on the target number of controls to be filled and preset arrangement orders. Colors of adjacent color blocks of the target number of color blocks with the preset arrangement orders are most similar.

In some embodiment of the present disclosure, the target number of color blocks include a first color block, the target number of controls to be filled include a first control to be filled, the first control to be filled is filled with the first color block, and after the one of the target number of color blocks is filled to one of the controls to be filled in response to the selection operation acting on the one of the target number of color blocks, the method includes: in response to detecting a cancellation operation acting on the first color block on the first control to be filled, the filling of the first color block on the first control to be filled is removed and the first control to be filled is kept in a filling vacancy state.

In some embodiment of the present disclosure, in response to detecting a cancellation operation acting on the first color block on the first control to be filled, the filling of the first color block on the first control to be filled is removed and the first control to be filled is kept in a filling vacancy state, including: in response to detecting the cancellation operation acting on the first color block on the first control to be filled, the first color block is moved from the first control to be filled to the first region, or the first color block is moved from the first control to be filled to a second control to be filled. The second control to be filled is any other control to be filled in the target number of controls to be filled except for the first control to be filled.

In some embodiment of the present disclosure, in response to detecting the cancellation operation acting on the first color block on the first control to be filled, the first color block is moved from the first control to be filled to the first region, including: in response to detecting a click operation acting on the first color block on the first control to be filled, the first color block is moved from the first control to be filled to the first region.

In some embodiment of the present disclosure, in response to detecting a click operation acting on the first color block on the first control to be filled, the first color block is moved from the first control to be filled to the first region, including: in response to detecting the click operation acting on the first color block on the first control to be filled, the first color block is moved from the first control to be filled to an initial display position in the first region; or in response to detecting the click operation acting on the first color block on the first control to be filled, the first color block is moved from the first control to be filled to any vacant position in the first region.

In some embodiment of the present disclosure, in response to detecting the cancellation operation acting on the first color block on the first control to be filled, the first color block is moved from the first control to be filled to the first region, or the first color block is moved from the first control to be filled to a second control to be filled, including: in response to detecting a drag operation acting on the first color block on the first control to be filled, a drag position corresponding to the drag operation is obtained; in response to the drag position exceeding a preset range, the first color block is moved from the first control to be filled to the first region; or, in response to the drag position not exceeding the preset range, the first color block is moved from the first control to be filled to the second control to be filled.

In some embodiment of the present disclosure, in response to the drag position not exceeding the preset range, the first color block is moved from the first control to be filled to the second control to be filled, including: in response to the drag position not exceeding the preset range, a control to be filled closest to the drag position from the target number of controls to be filled is determined as the second control to be filled; and the first color block is moved from the first control to be filled to the second control to be filled.

In some embodiment of the present disclosure, the method includes: in response to detecting the drag operation acting on the first color block on the first control to be filled, the first color block is highlighted; and in response to detecting that the drag operation acting on the first color block on the first control to be filled is ended, a normal display of the first color block is restored.

In some embodiment of the present disclosure, based on a relationship between arrangement orders of the target number of color blocks on the target number of controls to be filled and preset arrangement orders, display colors of the electronic device are adjusted, including: in response to the arrangement orders being consistent with the preset arrangement orders, attribute information of a user corresponding to the electronic device is acquired and the display colors of the electronic device are adjusted based on the attribute information; or, in response to the arrangement orders being inconsistent with the preset arrangement orders, color blocks whose arrangement orders are inconsistent with the preset arrangement orders from the target number of color blocks are determined as second color blocks and the display colors of the electronic device are adjusted based on the second color blocks.

In some embodiment of the present disclosure, in response to the arrangement orders being inconsistent with the preset arrangement orders, color blocks whose arrangement orders are inconsistent with the preset arrangement orders from the target number of color blocks are determined as second color blocks and the display colors of the electronic device are adjusted based on the second color blocks, including: in response to the arrangement orders being inconsistent with the preset arrangement orders, the color blocks whose arrangement orders are inconsistent with the preset arrangement orders from the target number of color blocks are determined as the second color blocks and a number of the second color blocks is obtained; weight analysis is performed on the second color blocks and the number of the second color blocks to obtain an analysis result; and based on the analysis result, the display colors of the electronic device are adjusted.

In some embodiment of the present disclosure, the method includes the target number of color blocks are displayed and the second color blocks are highlighted.

In some embodiment of the present disclosure, the electronic device includes a touch screen, and the displaying a color testing interface includes: in response to an instruction to enter the color testing interface, the touch screen is calibrated to a uniform color; and the color testing interface on the calibrated touch screen is displayed.

In some embodiment of the present disclosure, the color testing interface is displayed, including: a color testing selection interface is displayed, the color testing selection interface includes a first testing selection control and a second testing selection control, where the target number of color blocks corresponding to a testing mode to which the first testing selection control belongs is less than the target number of color blocks corresponding to a testing mode to which the second testing selection control belongs; in response to detecting a touch operation on the first testing selection control, a first color testing interface is displayed; in response to detecting a touch operation on the second testing selection control, a second color testing interface is displayed.

In some embodiment of the present disclosure, when the first color testing interface is displayed, the target number of color blocks are displayed through a current interface corresponding to the first region, and when the second color testing interface is displayed, the target number of color blocks are displayed through a plurality of consecutive interfaces corresponding to the first region.

In some embodiment of the present disclosure, the testing mode to which the first testing selection control belongs corresponds to 16 color blocks, and the testing mode to which the second testing selection control belongs corresponds to 42 color blocks.

In some embodiment of the present disclosure, the method includes: a current control to be filled from the target number of controls to be filled is determined during a process of filling the target number of color blocks into the target number of controls to be filled; and the current control to be filled is highlighted.

In some embodiment of the present disclosure, after the color testing interface is displayed, the method includes: in response to detecting a cancellation testing operation acting on the color testing interface, the color testing interface is exited.

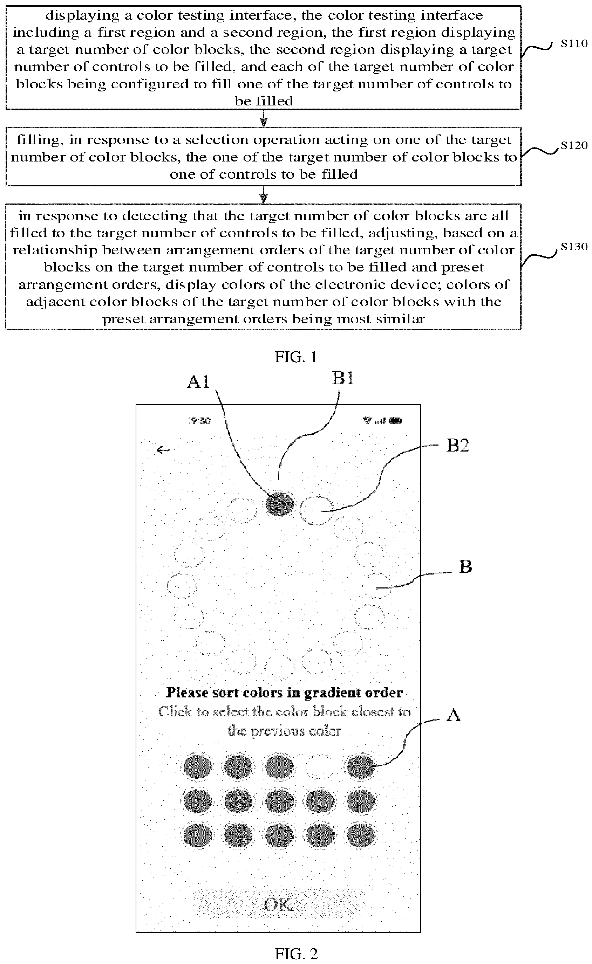

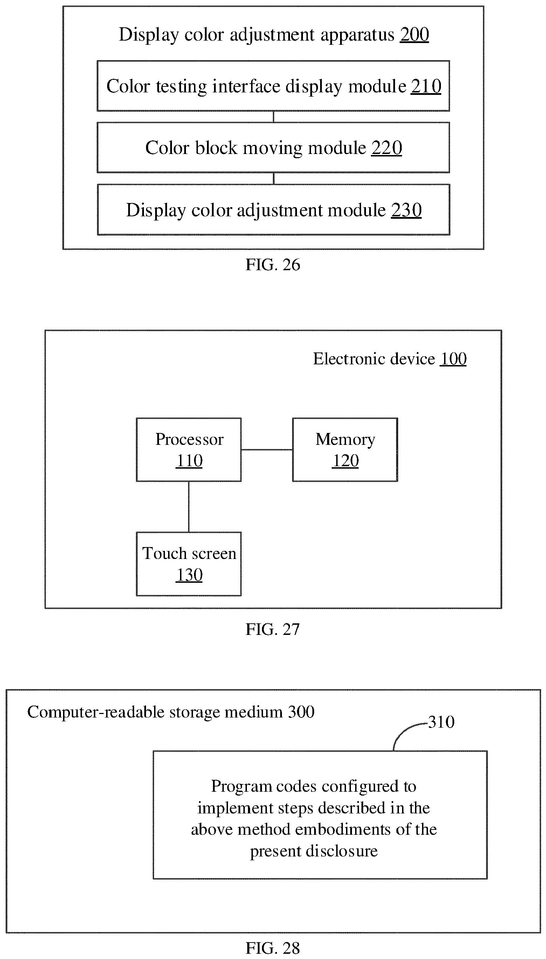

As illustrated in FIG. 1, FIG. 1 illustrates a schematic flowchart of a method for adjusting display colors according to an embodiment of the present disclosure. The method for adjusting display colors is used to provide a color testing interface for sorting similar colors and adjust the display colors of the electronic device based on the test results, the display colors are personalized to enhance the user experience. In specific embodiments, the method for adjusting display colors is implemented by an apparatus for adjusting display colors 200 illustrated in FIG. 26 and an electronic device 100 configured with the apparatus for adjusting display colors 200 (FIG. 27). In the following, an electronic device is taken as an example to describe the specific process of this embodiment. Of course, it is understandable that the electronic device applied in this embodiment may be a smart phone, a tablet computer, a wearable electronic device, etc., and is not specifically limited herein. The process illustrated in FIG. 1 will be described in detail below, and the method for adjusting display colors includes a block S 110 through a block S 130 as follows.

At the block S 110: displaying a color testing interface, the color testing interface including a first region and a second region, the first region displaying a target number of color blocks, the second region displaying a target number of controls to be filled, and each of the target number of color blocks being configured to fill one of the target number of controls to be filled.

In this embodiment, when the user wants to enter the color testing interface of the electronic device, the user first needs to send an instruction to the electronic device to instruct the electronic device to turn on and display the color testing interface. The instructions may be generated by the user through performing a touch operation on an application icon corresponding to the color testing interface set on an interface of the electronic device, or by voice generation when the user inputs an instruction to enter the color testing interface on the electronic device, which is not specifically limited herein. As one way, the user may instruct the electronic device to enter and display the color testing interface by clicking on the "Settings-Visual enhancement" of the electronic device.

In some embodiments, the color testing interface includes a first region and a second region, the first region may be disposed above the second region, below the second region, to the left of the second region, to the right of the second region, or around the surrounding of the second region. Specifically, in this embodiment, the first region is disposed above the second region. In an embodiment, a size of the first region may be greater than a size of the second region, equal to the size of the second region, or smaller than the size of the second region. Specifically, in this embodiment, the size of the first region is greater than the size of the second region. Of course, there are other positional and dimensional relationships between the first region and the second region, which are not exhaustive herein.

In some embodiments, when the electronic device displays the color testing interface, the touch screen may be calibrated to a uniform color, so that the brightness, color temperature, and color gamut of the touch screen are at the same level for visual feature testing. For example, when the brightness, color temperature, and color gamut of the touch screen are at the same level, the D15 color vision test (basic visual feature test method) or the Munsell test (professional visual feature test method) with scientific theoretical support is selected to test the visual features of different users.

FIG. 2 illustrates a schematic diagram of a first kind of interface of the electronic device according to an embodiment of the present disclosure. As illustrated in FIG. 2, in some embodiments, before the electronic device displays the color testing interface, a color testing transition interface may also be displayed. The color testing transition interface may include a "Start" control, and when a touch operation acting on the "Start" control is detected, the color testing interface may be entered and displayed.

In some embodiments, the first region of the color testing interface displays a target number of color blocks, the second region displays a target number of controls to be filled, and each color block is used to fill one of the target number of controls to be filled. The shape of the color block and the shape of the control to be filled may be the same or different, which is not limited herein.

Taking D15 as an example, 16 color blocks are used for visual test corresponding to D15 testing scheme, which is the basic color chess testing scheme and can qualitatively determine the user's visual ability including visually impaired red blind (or color weakness), green blind (or color weakness), blue blind (or color weakness), full color blindness, and medium vision of normal users. Through the weight analysis of the test results, a set of scoring system is obtained, which correspondingly gives users a set of exclusive color compensation scheme, gives the ability to discriminate colors for people with impairments, and gives color preferences to users with normal vision. Therefore, when using the D15 testing scheme, the first region can display 16 color blocks, and the second region can display 16 controls to be filled.

Taking Munsell as an example, corresponding to the traditional 94 Munsell testing scheme, a simplified version is made on the traditional scheme, with 42 color chesses selected to achieve the testing scheme on the electronic device. The Munsell testing scheme is more professional in both qualitative and quantitative abilities compared to the D15 testing scheme, and the granularity of the testing scheme is more accurate, hence it is called a professional visual feature test. Therefore, when using the Munsell testing scheme, the first region can display 42 color blocks, and the second region can display 42 controls to be filled.

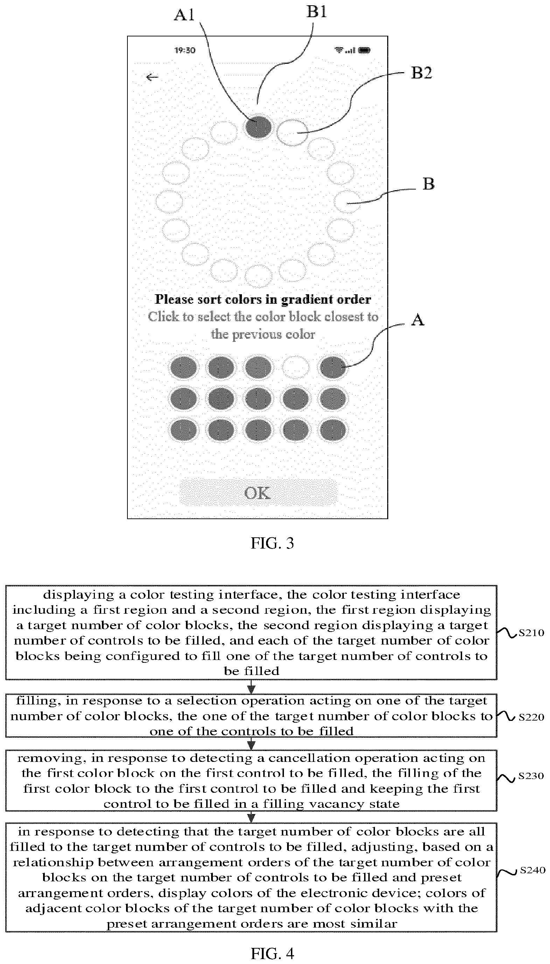

FIG. 3 illustrates a schematic diagram of a second kind of interface of the electronic device according to an embodiment of the present disclosure. As illustrated in FIG. 3, taking the target number of color blocks as 16 color blocks and the target number of controls to be filled as 16 controls to be filled as examples, in some embodiments, the electronic device can display 16 color blocks A in the first region and 16 controls to be filled B in the second region, and one color block A1 of the 16 color blocks A has been filled to one control to be filled B 1 of the 16 controls to be filled B.

At the block S 120: filling, in response to a selection operation acting on one of the target number of color blocks, the one of the target number of color blocks to one of the controls to be filled.

In this embodiment, the electronic device can detect the selection operation acting on the color block during the display of the color testing interface, and fill the color block indicated by the selection operation to the corresponding control to be filled. In some embodiments, for the target number of color blocks, when the electronic device detects a selection operation acting on any color block in the target number of color blocks, the electronic device may fill the color block indicated by the selection operation into the corresponding control to be filled. For example, the target number of color blocks may include a first color block, the target number of controls to be filled may include a first control to be filled, and the electronic device may detect the selection operation acting on the first color block during the display of the color testing interface, and fill the first color block to the first control to be filled when the selection operation acting on the first color block is detected.

In some embodiments, assuming that the target number of color blocks include color block 1, color block 2, color block 3, and color block 4, the target number of controls to be filled include control to be filled 1, control to be filled 2, control to be filled 3, and control to be filled 4, and the target number of controls to be filled are sequentially filled in the order of the control to be filled 1, the control to be filled 2, the control to be filled 3, and the control to be filled 4, when the selection operation acting on the color block 2 is first detected, the color block 2 may be filled into the control to be filled 1, when the selection operation acting on the color block 3 is subsequently detected, the color block 3 may be filled into the control to be filled 2, when the selection operation acting on the color block 1 is detected later, the color block 1 may be filled into the control to be filled 3, and when the selection operation acting on the color block 4 is finally detected, the color block 4 may be filled to the control to be filled 4. Thus, for each selection operation detected on one color block, one color block is filled to one control to be filled, and the above steps are repeated until the target number of color blocks are filled to the target number of controls to be filled.

As one way, during the process of filling the color block into the control to be filled, a current control to be filled may be determined from the target number of controls to be filled, and the current control to be filled may be highlighted, so that the user knows which the control to be filled corresponds to the currently selected color block. In some embodiments, during the process of filling the color block into the control to be filled, the current control to be filled may be determined sequentially from the target number of controls to be filled according to arrangement orders of the controls to be filled set by the electronic device, and the current control to be filled may be highlighted. In some embodiments, an unfilled control to be filled closest to a starting point in the target number of controls to be filled may be determined as the current control to be filled. As one way, highlighting the current control to be filled may include: lighting the current control to be filled, enlarging the current control to be filled, flashing the current control to be filled, etc., which is not limited herein. For example, as illustrated in FIG. 3, the current control to be filled B2 may be highlighted.

In some embodiments, the selection operation may include a click operation, a drag operation, etc., which is not limited here. Specifically, taking the selection operation as a click operation as an example, the electronic device may fill the first color block into the first control to be filled when detecting the click operation acting on the first color block. Taking the selection operation as a drag operation as an example, the electronic device may move the first color block along a dragging track and fill the first color block to the first control to be filled when detecting the drag operation acting on the first color block.

In some embodiments, the color block is filled to the control to be filled may include: the color block is moved to the control to be filled for filling and display, and the display of the color block in the first region is cancelled. Alternatively, the color block is copied to the control to be filled for filling and display, and the display of the color block in the first region is maintained, which is not limited herein.

At the block S 130: in response to detecting that the target number of color blocks are all filled to the target number of controls to be filled, adjusting, based on a relationship between arrangement orders of the target number of color blocks on the target number of controls to be filled and preset arrangement orders, display colors of the electronic device; colors of adjacent color blocks of the target number of color blocks with the preset arrangement orders being most similar.

In this embodiment, the electronic device may detect whether the target number of color blocks are all filled to the target controls to be filled, if it is detected that the target number of color blocks are all filled to the target number of controls to be filled, the arrangement orders of the target number of color blocks on the target number of controls to be filled may be obtained. In some embodiments, it may be detected whether the target number of controls to be filled are all filled. When it is detected that the target number of controls to be filled are all filled, it may be determined that the target number of color blocks are all filled to the target number of controls to be filled, otherwise, it may be determined that the target number of color blocks are not all filled to the target number of controls to be filled.

As one way, when it is detected that some color blocks in the target number of color blocks are filled to some controls to be filled in the target number of controls to be filled, the arrangement orders of the some color blocks on the some controls to be filled may be obtained. As another way, when it is detected that the target number of color blocks are all filled to the target number of controls to be filled, the arrangement orders of the target number of color blocks on the target number of controls to be filled may be directly obtained. As still another way, when it is detected that the target number of color blocks are all filled to the target number of controls to be filled, an "OK" control may be displayed, and when a touch operation acting on the "OK" control is detected, the arrangement orders of the target number of color blocks on the target number of controls to be filled may be obtained. Before detecting the touch operation acting on the "OK" control, the arrangement orders of the target number of color blocks filled on the target number of controls to be filled may also be modified. For example, the touch operation acting on the first color block filled on the first control to be filled may also be received, and the filling of the first color block on the first control to be filled may be removed in response to the touch operation.

In some embodiments, during the process of filling the target number of color blocks to the target number of controls to be filled, if a cancellation testing operation acting on the color testing interface is detected when filling some color blocks to some controls to be filled, the color testing interface may be exited, which is not repeated herein.

In some embodiments, the electronic device may be preset and stored with preset arrangement orders, which are used as a basis for determining the arrangement orders of the target number of color blocks on the target number of controls to be filled. Therefore, in this embodiment, after obtaining the arrangement orders, the arrangement orders may be compared with the preset arrangement orders to obtain the relationship between the arrangement orders and the preset arrangement orders, and the colors of the electronic device are adjusted based on the relationship between the arrangement orders and the preset arrangement orders.

In some embodiments, the colors of adjacent color blocks of the target number of color blocks with the preset arrangement orders are most similar. Therefore, when the colors of the adjacent color blocks of the target number of color blocks with the arrangement orders are most similar, it may be considered that the arrangement orders are consistent with the preset arrangement orders, and the display colors of the electronic device may be adjusted in a manner that the arrangement orders are consistent with the preset arrangement orders. When the colors of the adjacent color blocks of the target number of color blocks with the arrangement orders are not most similar, it may be considered that the arrangement orders are inconsistent with the preset arrangement orders, and the display colors of the electronic device may be adjusted in a manner that the arrangement orders are inconsistent with preset arrangement orders.

For example, it is assumed that the target number of color blocks include color block 1, color block 2, color block 3, color block 4, and color block 5, and the preset arrangement orders are color block 1 - color block 3 - color block 5 - color block 4 - color block 1, that is, the color of the color block 3 is the most similar to the colors of the color block 1 and the color block 5, and the color of the color block 4 is the most similar to the colors of the color block 5 and the color block 1. When the arrangement orders of the color block 1, the color block 2, the color block 3, the color block 4, and the color block 5 are color block 1 - color block 3 - color block 5 - color block 4 - color block 1, it may be determined that the arrangement orders are consistent with the preset arrangement orders, otherwise, it may be determined that the arrangement orders are inconsistent with the preset arrangement orders.

The method for adjusting display colors according to the embodiment of the present disclosure displays the color testing interface. The color testing interface includes the first region and the second region, the first region displays the target number of color blocks, the second region displays the target number of controls to be filled, and each color block is used to fill one control to be filled. In response to the selection operation acting on the color block, the color blocks are filled into the controls to be filled. When it is detected that the target number of color blocks are all filled into the target number of controls to be filled, based on the relationship between the arrangement orders of the target number of color blocks on the target number of controls to be filled and the preset arrangement orders, the display colors of the electronic device are adjusted. The colors of the adjacent color blocks of the target number of color blocks with the preset arrangement orders are most similar. Thus, by providing the color testing interface for sorting similar colors and adjusting the display colors of the electronic device based on the test results, personalized display colors can be achieved and the user experience can be improved.

FIG. 4 illustrates a schematic flowchart of a method for adjusting display colors according to another embodiment of the present disclosure. The method is implemented by the electronic device mentioned above. The process illustrated in FIG. 4 will be described below in detail. In this embodiment, the target number of color blocks include a first color block, the target number of controls to be filled include a first control to be filled, and the first control to be filled is filled with the first color block. The method for adjusting display colors may specifically include a block S210 through a block S240 as follows.

At the block S210: displaying a color testing interface, the color testing interface including a first region and a second region, the first region displaying a target number of color blocks, the second region displaying a target number of controls to be filled, and each of the target number of color blocks being configured to fill one of the target number of controls to be filled.

At the block S220: filling, in response to a selection operation acting on one of the target number of color blocks, the one of the target number of color blocks to one of the controls to be filled.

In some embodiments, the specific description of the blocks S210-S220 may be found in the blocks S 110-S 120, which will not be repeated herein.

At the block S230: removing, in response to detecting a cancellation operation acting on the first color block on the first control to be filled, the filling of the first color block to the first control to be filled and keeping the first control to be filled in a filling vacancy state.

In some embodiments, after filling the first color block to the first control to be filled, when the cancellation operation acting on the first color block on the first control to be filled is detected, the filling of the first color block on the first control to be filled is removed to provide the user with modifications to the filling of the color block. In some embodiments, the cancellation operation may include: a click operation, a drag operation, etc., which is not limited herein.

In some embodiments, after filling the first color block to the first control to be filled, when the cancellation operation acting on the first color block on the first control to be filled is detected, the filling the first color block on the first control to be filled is removed and the first control to be filled is kept in a display vacancy state. As one way, during the process of keeping the first control to be filled in the display vacancy state, the first control to be filled can be highlighted, for example, the first control to be filled may be lighted, enlarged to display the first control to be filled, flashed to display the first control to be filled, etc., which is not limited herein.

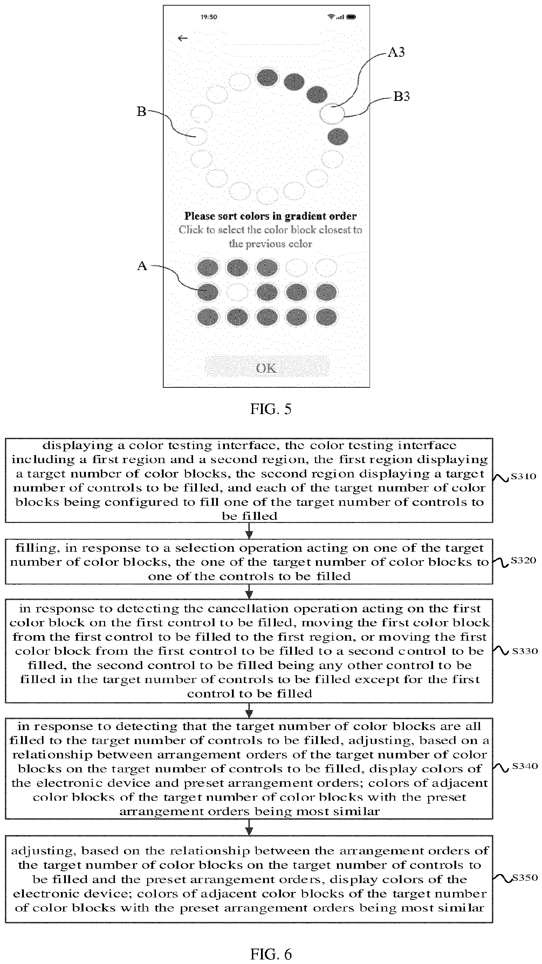

FIG. 5 illustrates a schematic diagram of a third kind of interface of the electronic device according to an embodiment of the present disclosure. As illustrated in FIG. 5, taking the target number of color blocks as 16 color blocks and the target number of controls to be filled as 16 controls to be filled as examples, in some embodiments, when the electronic device detects the cancellation operation acting on the first color block A3 on the first control to be filled B3, the electronic device removes the filling on the first control to be filled B3 and keeps the first control to be filled B3 in a display vacancy state. In addition, the first control to be filled B3 may also be highlighted.

At the block S240: in response to detecting that the target number of color blocks are all filled to the target number of controls to be filled, adjusting, based on a relationship between arrangement orders of the target number of color blocks on the target number of controls to be filled and preset arrangement orders, display colors of the electronic device; colors of adjacent color blocks of the target number of color blocks with the preset arrangement orders are most similar.

In some embodiments, the specific description of the blocks S240-S250 may be found in the blocks S 130-S 140, which will not be repeated herein.

The method for adjusting display colors according to the another embodiment of the present disclosure displays the color testing interface. The color testing interface includes the first region and the second region, the first region displays the target number of color blocks, the second region displays the target number of controls to be filled, and each color block is used to fill one control to be filled. In response to the selection operation acting on the color block, the color blocks are filled into the controls to be filled. When the cancellation operation acting on the first color block on the first control to be filled is detected, the filling of the first color block on the first control to be filled is removed and the first control to be filled is kept in the filling vacancy state. When it is detected that the target number of color blocks are all filled to the target number of controls to be filled, the display colors of the electronic device are adjusted based on the relationship between the arrangement orders of the target number of color blocks on the target number of controls to be filled and the preset arrangement orders. The colors of the adjacent color blocks of the target number of color blocks with the preset arrangement orders are most similar. Compared to the method for adjusting display colors illustrated in FIG. 1, this embodiment further detects the cancellation operation acting on the color block on the control to be filled, and removes the filling of the color block on the control to be filled and keeps the control to be filled in the vacancy state to improve the user's interaction experience.

FIG. 6 illustrates a schematic flowchart of a method for adjusting display colors according to still another embodiment of the present disclosure. The method is implemented by to the electronic device mentioned above. The process illustrated in FIG. 6 will be described below in detail. In this embodiment, the target number of color blocks include a first color block, the target number of controls to be filled include a first control to be filled, and the first control to be filled is filled with the first color block. The method for adjusting display colors may specifically include a block S310 through a block S350 as follows.

At the block S310: displaying a color testing interface, the color testing interface including a first region and a second region, the first region displaying a target number of color blocks, the second region displaying a target number of controls to be filled, and each of the target number of color blocks being configured to fill one of the target number of controls to be filled.

At the block S320: filling, in response to a selection operation acting on one of the target number of color blocks, the one of the target number of color blocks to one of the controls to be filled.

In some embodiments, the specific description of the blocks S310-S320 may be found in the blocks S110-S120, which will not be repeated herein.

At the block S330: in response to detecting the cancellation operation acting on the first color block on the first control to be filled, moving the first color block from the first control to be filled to the first region, or moving the first color block from the first control to be filled to a second control to be filled, the second control to be filled being any other control to be filled in the target number of controls to be filled except for the first control to be filled.

As one way, after filling the first color block into the first control to be filled, when the cancellation operation acting on the first color block on the first control to be filled is detected, the first color block may be moved from the first control to be filled to the first region. As another way, after filling the first color block into the first control to be filled, when the cancellation operation acting on the first color block on the first control to be filled is detected, the first color block may be moved from the first control to be filled to the second control to be filled, where the second control to be filled is any other control to be filled in the target number of controls to be filled except for the first control to be filled.

In some embodiments, after filling the first color block into the first control to be filled, when a click operation acting on the first color block on the first control to be filled is detected, the first color block may be moved from the first control to be filled to the first region. As one way, the first color block may be moved from the first control to be filled to an initial display position in the first region, or the first color block may be moved from the first control to be filled to any vacant position in the first region, which is not specifically limited herein.

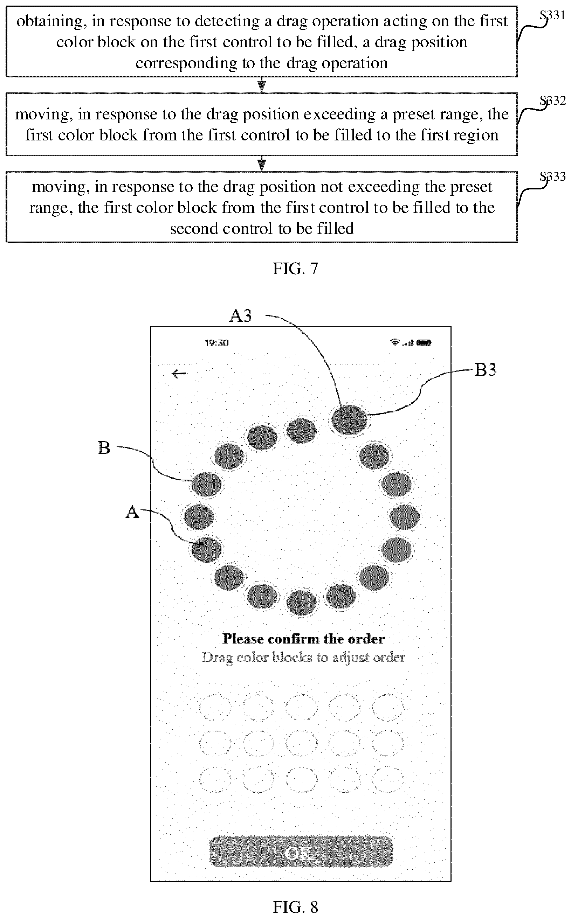

FIG. 7 illustrates a schematic flowchart of the block S330 of the method for adjusting display colors illustrated in FIG. 6 of the present disclosure. The process illustrated in FIG. 7 will be described below in detail, which may include a block S331 through a block S333 as follows.

At the block S331: obtaining, in response to detecting a drag operation acting on the first color block on the first control to be filled, a drag position corresponding to the drag operation.

In some embodiments, when a drag operation acting on the first color block on the control to be filled is detected, the corresponding drag position corresponding to the drag operation may be obtained. Specifically, the drag position corresponding to the drag operation may be detected through contact sensors or pressure sensors disposed on the touch screen.

In some embodiments, when a drag operation acting on the first color block on the first control to be filled is detected, the first color block may be highlighted, and when it is detected that the drag operation acting on the first color block on the first control to be filled is ended, the normal display of the first color block may be restored. For example, when the drag operation acting on the first color block on the first control to be filled is detected, the first color block may be lighted, enlarged to display the first color block, flashed to display the first color block, etc. When it is detected that the drag operation acting on the first color block on the first control to be filled is ended, the highlighting, enlarging in to display, flashing to display, etc. of the first color block may be canceled, which is no limited herein.

FIG. 8 illustrates a schematic diagram of a fourth kind of interface of the electronic device according to an embodiment of the present disclosure. As illustrated in FIG. 8, taking the target number of color blocks as 16 color blocks and the target number of controls to be filled as 16 controls to be filled as examples, when a drag operation acting on the first color block A3 on the first control to be filled B3 is detected, the first color block A3 may be enlarged and displayed.

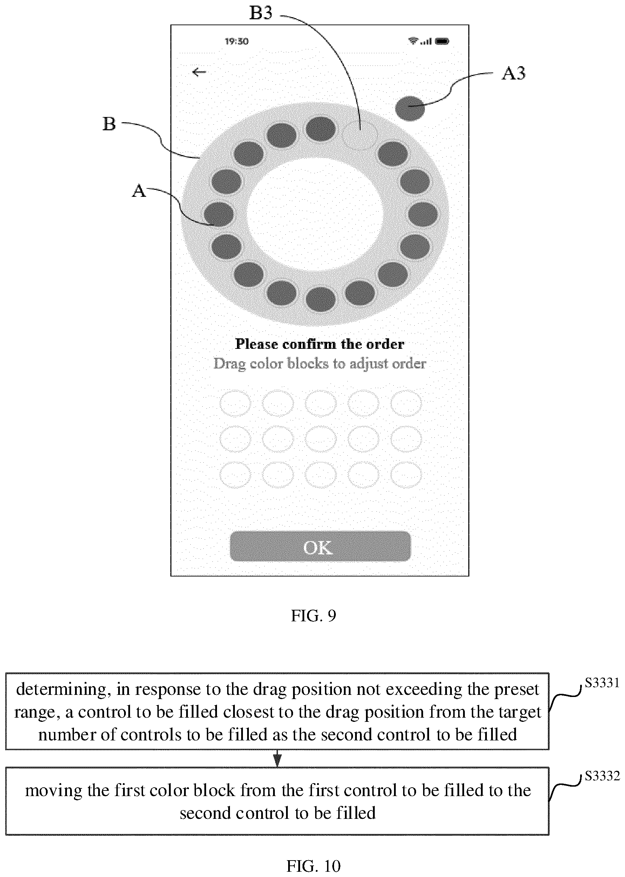

FIG. 9 illustrates a schematic diagram of a fifth kind of interface of the electronic device according to an embodiment of the present disclosure. As illustrated in FIG. 9, taking the target number of color blocks as 16 color blocks and the target number of controls to be filled as 16 controls to be filled as examples, when it is detected that the drag operation acting on the first color block A3 on the first control to be filled B3 is ended, the normal display of the first color block A3 may be restored.

At the block S332: moving, in response to the drag position exceeding a preset range, the first color block from the first control to be filled to the first region.

In some embodiments, the electronic device may be preset and stored with a preset range, which is used as a basis for determining the drag position. Therefore, in this embodiment, when obtaining the drag position corresponding to the drag operation, the drag position may be compared with the preset range to determine whether the drag position exceeds the preset range.

In some embodiments, when the drag position exceeds the preset range, the first color block may be moved from the first control to be filled to the first region. As one way, when the drag position exceeds the preset range and the drag operation ends, the first color block may be moved from the first control to be filled to the first region.

At the block S333: moving, in response to the drag position not exceeding the preset range, the first color block from the first control to be filled to the second control to be filled.

In some embodiments, when the drag position does not exceed the preset range, the first color block may be moved from the first control to be filled to the second control to be filled. As one way, when the drag position does not exceed the preset range and the drag operation ends, the first color block may be moved from the first control to be filled to the second control to be filled.

FIG. 10 illustrates a schematic flowchart of the block S333 of the method for adjusting display colors illustrated in FIG. 6 of the present disclosure. The process illustrated in FIG. 10 will be described below in detail, which may include a block S3331 through a block S3332.

At the block S3331: determining, in response to the drag position not exceeding the preset range, a control to be filled closest to the drag position from the target number of controls to be filled as the second control to be filled.

At the block S3332: moving the first color block from the first control to be filled to the second control to be filled.

In this embodiment, when the drag position does not exceed the preset range, the control to be filled closest to the drag position may be determined from the target number of controls to be filled as the second control to be filled, and the first color block may be moved from the first control to be filled to the second control to be filled. In some embodiments, position information of each control to be filled in the target number of controls to be filled may be obtained, based on the position information of each control to be filled and position information of the drag position, the control to be filled closest to the drag position may be determined from the target number of controls to be filled as the second control to be filled. For example, the position coordinate of each control to be filled in the target number of controls to be filled may be obtained, based on the position coordinate of each control to be filled and the position coordinate of the drag position, the distance from the drag position to each control to be filled may be calculated, and the control to be filled closest to the drag position may be determined from the target number of controls to be filled as the second control to be filled.

At the block S340: in response to detecting that the target number of color blocks are all filled to the target number of controls to be filled, adjusting, based on a relationship between arrangement orders of the target number of color blocks on the target number of controls to be filled and preset arrangement orders, display colors of the electronic device; colors of adjacent color blocks of the target number of color blocks with the preset arrangement orders are most similar.

In some embodiments, the specific description of the block S340 may be found in the block S 130, which will not be repeated herein.

The method for adjusting display colors according to the still another embodiment of the present disclosure displays the color testing interface. The color testing interface includes the region first and the second region, the first region displays the target number of color blocks, the second region displays the target number of controls to be filled, and each color block is used to fill one control to be filled. In response to the selection operation acting on the color block, the color block is filled into the control to be filled. In the process of filling the target number of color blocks to the target number of controls to be filled, when the cancellation operation acting on the first color block on the first control to be filled is detected, the first color block is moved from the first control to be filled to the first region, or the first color block is moved from the first control to be filled to the second control to be filled, where the second control to be filled is any other control to be filled in the target number of controls to be filled except for the first control to be filled. When it is detected that the target number of color blocks are all filled to the target number of controls to be filled, the display colors of the electronic device are adjusted based on the relationship between the arrangement orders of the target number of color blocks on the target number of controls to be filled and the preset arrangement orders, where the colors of the adjacent color blocks formed by the target number of color blocks in the preset arrangement order are most similar. Compared to the method for adjusting display colors illustrated in FIG. 1, this embodiment also moves the color block to the first region or the other control to be filled when the cancellation operation acting on the color block on the control to be filled is detected to improve the user's interaction experience.

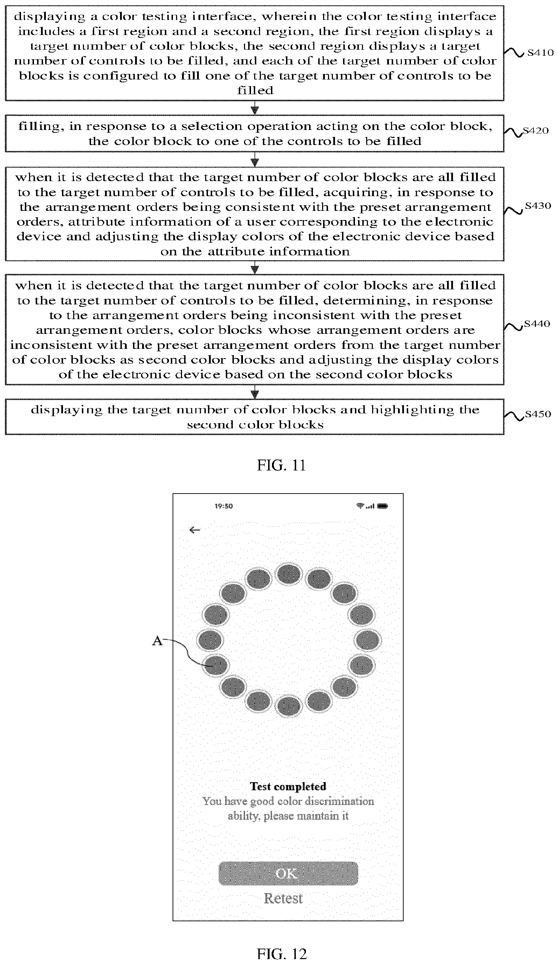

FIG. 11 illustrates a schematic flowchart of a method for adjusting display colors according to even still another embodiment of the present disclosure. The method is implemented by the electronic device mentioned above. The process illustrated in FIG. 11 will be described below in detail. The method for adjusting display colors may include a block S410 through a block S450 as follows.

At the block S410: displaying a color testing interface, the color testing interface including a first region and a second region, the first region displaying a target number of color blocks, the second region displaying a target number of controls to be filled, and each of the target number of color blocks being configured to fill one of the target number of controls to be filled.

At the block S420: filling, in response to a selection operation acting on the color block, the color block to one of the controls to be filled.

In some embodiments, the specific description of the blocks S410-S420 may be found in the blocks S 110-S 120, which will not be repeated herein.

At the block S430: when it is detected that the target number of color blocks are all filled to the target number of controls to be filled, acquiring, in response to the arrangement orders being consistent with the preset arrangement orders, attribute information of a user corresponding to the electronic device and adjusting the display colors of the electronic device based on the attribute information.

In some embodiments, when the arrangement orders are consistent with the preset arrangement orders, it indicates that the user sorts all the target number of color blocks correctly, then the user has strong color identification ability and color compensation can be given to such user for color preferences according to the user's preferences. Therefore, in this embodiment, when the arrangement orders are consistent with the preset arrangement orders, the attribute information of the user corresponding to the electronic device may be obtained, the user's preferences may be determined based on the user's attribute information, and the display colors of the electronic device can be adjusted based on the user's preferences to make the adjusted display colors more in line with the user's preferences.

In some embodiments, the user's attribute information may include age, gender, region, scope of activity, etc., which is not limited herein.

At the block S440: when it is detected that the target number of color blocks are all filled to the target number of controls to be filled, determining, in response to the arrangement orders being inconsistent with the preset arrangement orders, color blocks whose arrangement orders are inconsistent with the preset arrangement orders from the target number of color blocks as second color blocks and adjusting the display colors of the electronic device based on the second color blocks.

In some embodiments, when the arrangement orders are inconsistent with the preset arrangement orders, it indicates that the user does not have all the target number of color blocks correctly sorted, then the user has weak color identification ability and color compensation can be given to such user based on the user's amblyopia. Therefore, in this embodiment, when the arrangement orders are inconsistent with the preset arrangement orders, the color blocks that the arrangement orders are inconsistent with the preset arrangement orders may be determined from the target number of color blocks as the second color blocks, and the display colors of the electronic device may be adjusted based on the second color blocks. As one way, the second color blocks indicate that the user makes errors in sorting the color, then a weight analysis may be performed on the second color blocks in which the user makes errors and on the number of the second color blocks to obtain an analysis result, and the display colors of the electronic device are adjusted based on the analysis result.

At the block S450: displaying the target number of color blocks and highlighting the second color blocks.

In some embodiments, after obtaining the test result, the target number of color blocks may be displayed, and when the second color blocks exist in the target number of color blocks, the second color blocks are highlighted. For example, the second color blocks may be highlighted, enlarged, or flashed, which is not limited herein.

FIG. 12 illustrates a schematic diagram of a sixth kind of interface of the electronic device according to an embodiment of the present disclosure. As illustrated in FIG. 12, taking the target number of color blocks as 16 color blocks and the target number of controls to be filled as 16 controls to be filled as examples, when the arrangement orders are consistent with the preset arrangement orders, the target number of color blocks A may be displayed normally after obtaining the test result.

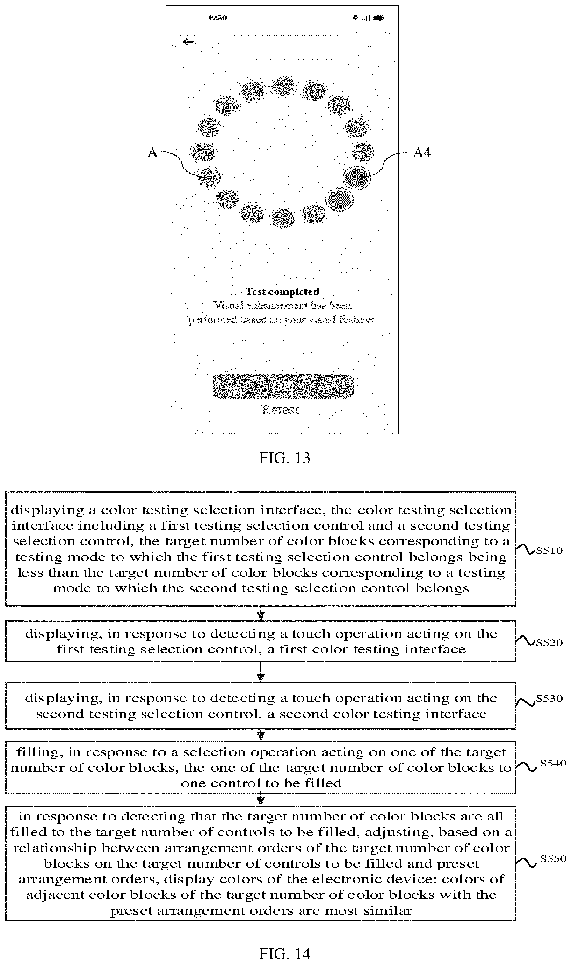

FIG. 13 illustrates a schematic diagram of a seventh kind of interface of the electronic device according to an embodiment of the present disclosure. As illustrated in FIG. 13, taking the target number of color blocks as 16 color blocks and the target number of controls to be filled as 16 controls to be filled as examples, when the arrangement orders are inconsistent with the preset arrangement orders, after obtaining the test result, the target number of color blocks A may be displayed and the second color blocks A4 in the target number of color blocks A may be highlighted.

The method for adjusting display colors according to the even still another embodiment of the present disclosure displays the color testing interface. The color testing interface includes the first region and the second region, the first region displays the target number of color blocks, the second region displays the target number of controls to be filled, and each color block is used to fill one control to be filled. In response to the selection operation acting on the color block, the color block is filled into the control to be filled. When it is detected that the target number of color blocks are all filled to the target number of controls to be filled, the attribute information of the user corresponding to the electronic device is obtained and the display colors of the electronic device are adjusted based on this attribute information when the arrangement orders of the target number of color blocks on the target number of controls to be filled are consistent with the preset arrangement orders. When it is detected that the target number of color blocks are all filled to the target number of controls to be filled, the color blocks whose arrangement orders are inconsistent with the preset arrangement order are determined from the target number of color blocks as the second color blocks and the display colors of the electronic device are adjusted based on the second color blocks when the arrangement orders of the target number of color blocks on the target number of controls to be filled are inconsistent with the preset arrangement orders. The target number of color blocks are displayed and the second color blocks are highlighted. Compared to the method for adjusting display colors illustrated in FIG. 1, different display colors are adjusted based on the consistency or inconsistency between the arrangement orders and the preset arrangement orders according to this embodiment, thereby to adapt to different users and improve their sensory experience. In addition, the color blocks with incorrect arrangement are highlighted in this embodiment, so that the user can intuitively observe the color blocks with incorrect arrangement, and the user's interaction experience is improved.

FIG. 14 illustrates a schematic flowchart of a method for adjusting display colors according to further still another embodiment of the present disclosure. This method is implemented by the electronic device mentioned above. The process illustrated in FIG. 14 will be described below in detail. The method for adjusting display colors may include a block S510 through a block S550 as follows.

At the block S510: displaying a color testing selection interface, the color testing selection interface including a first testing selection control and a second testing selection control, the target number of color blocks corresponding to a testing mode to which the first testing selection control belongs being less than the target number of color blocks corresponding to a testing mode to which the second testing selection control belongs.

In this embodiment, the electronic device can display the color testing selection interface, and the color testing selection interface includes the first testing selection control and the second testing selection control. The target number of color blocks corresponding to the testing mode to which the first testing selection control belongs is less than the target number of color blocks corresponding to the testing mode to which the second testing selection control belongs, so that the user can choose whether to perform color testing through the testing mode to which the first testing selection control belongs or through the testing mode to which the second testing selection control belongs.

In some embodiments, the testing mode to which the first testing selection control belongs may be the D15 testing mode. In this case, the testing mode to which the first testing selection control belongs corresponds to 16 color blocks. The testing mode to which the second testing selection control belongs may be the simplified version of the Munsell testing mode. In this situation, the testing mode to which the second testing selection control belongs corresponds to 42 color blocks.

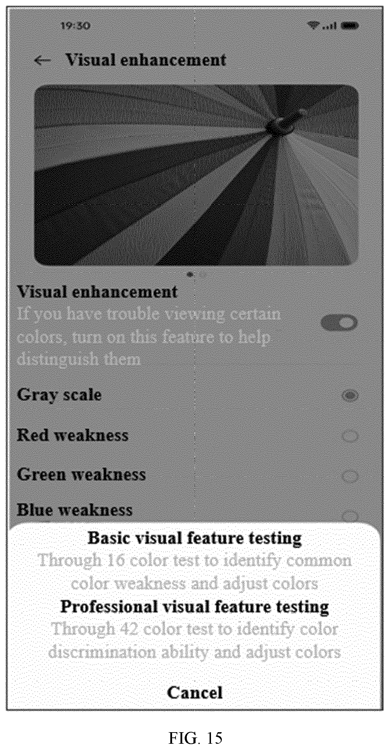

FIG. 15 illustrates a schematic diagram of an eighth kind of interface of the electronic device according to an embodiment of the present disclosure. As illustrated in FIG. 15, the electronic device may display a color testing selection interface, and the color testing selection interface includes the first testing selection control (basic visual feature testing) and the second testing selection control (professional visual feature testing).

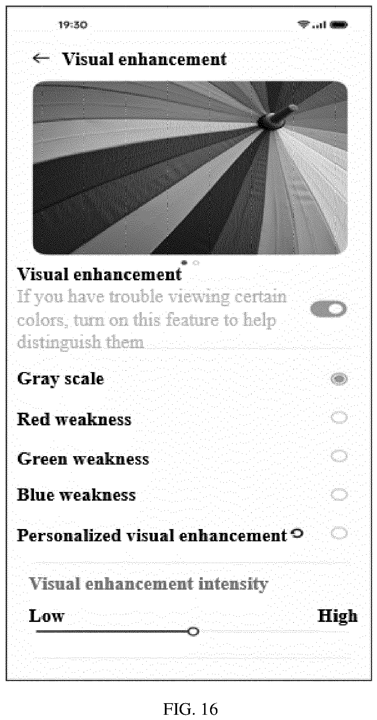

FIG. 16 illustrates a schematic diagram of a ninth kind of interface of the electronic device according to an embodiment of the present disclosure. As illustrated in FIG. 16, in some embodiments, before the electronic device displays the color testing selection interface, a color testing selection transition interface may also be displayed. The color testing selection transition interface may include a "Visual enhancement" control, and when a touch operation acting on the "Visual enhancement" control is detected, the color testing selection interface may be entered and displayed.

At the block S520: displaying, in response to detecting a touch operation acting on the first testing selection control, a first color testing interface.

In some embodiments, the electronic device may detect the touch operations acting on the first testing selection control and the second testing selection control during the display of the color testing selection interface. In this embodiment, when the touch operation acting on the first testing selection control is detected, the first color testing interface may be displayed. As one way, when the electronic device displays the first color testing interface, since the target number of color blocks are relatively small, the target number of color blocks may be displayed through the current interface corresponding to the first region, for example, 16 color blocks are displayed in the current interface of the first region.

At the block S530: displaying, in response to detecting a touch operation acting on the second testing selection control, a second color testing interface.

In this embodiment, when the touch operation acting on the second testing selection control is detected, the second color testing interface may be displayed. As one way, when the electronic device displays the second color testing interface, due to the large number of color blocks, the target number of color blocks may be displayed through multiple consecutive interfaces corresponding to the first region, and in this case, each interface of multiple consecutive interfaces respectively displays a part of the target number of color blocks. For example, 42 color blocks are divided into 4 groups, which are displayed on 4 consecutive interfaces corresponding to the first region, and the operation mode of each of the 4 groups is the same as the testing mode corresponding to the first testing selection control.

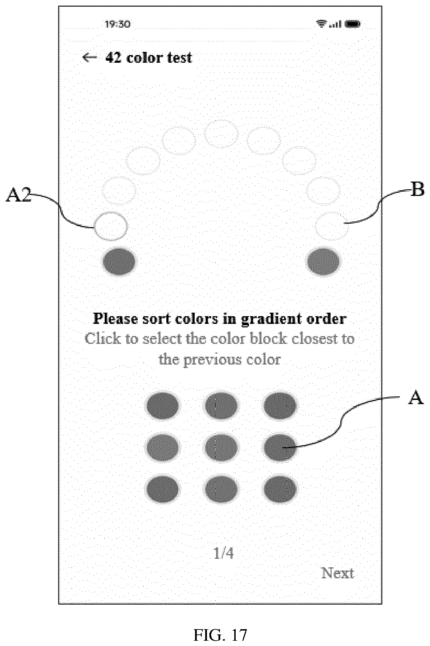

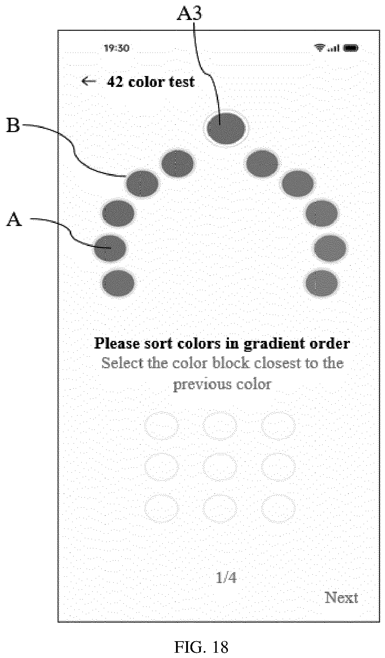

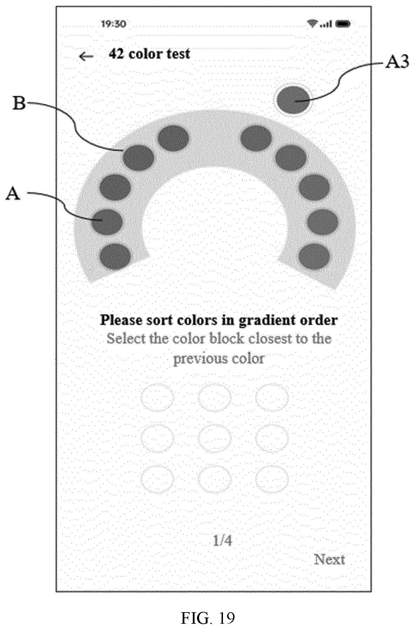

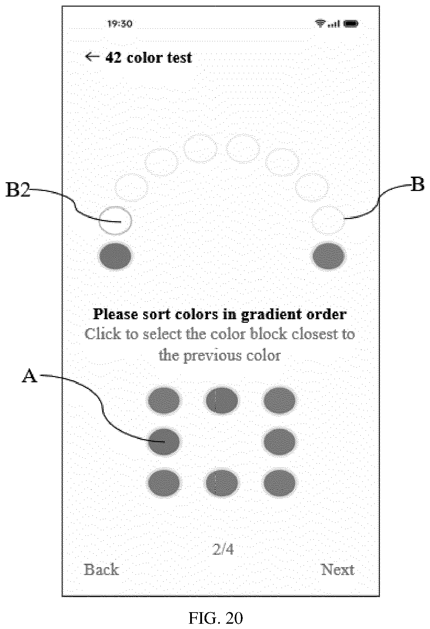

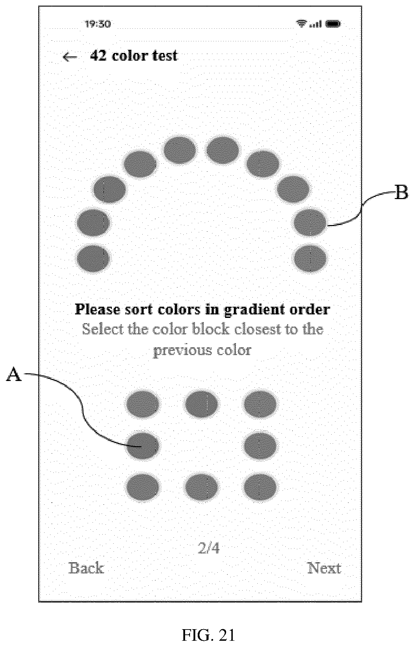

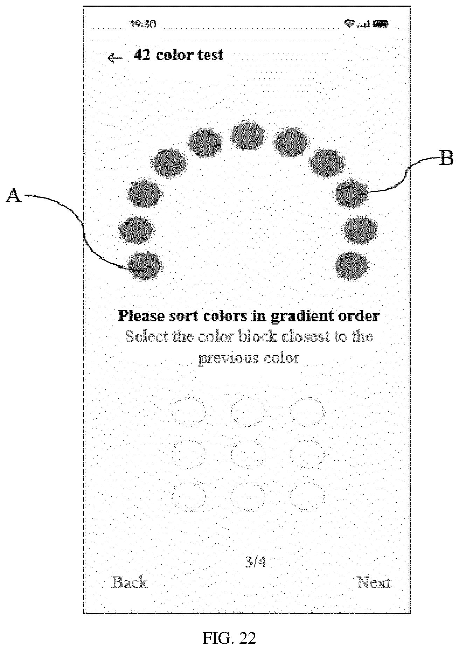

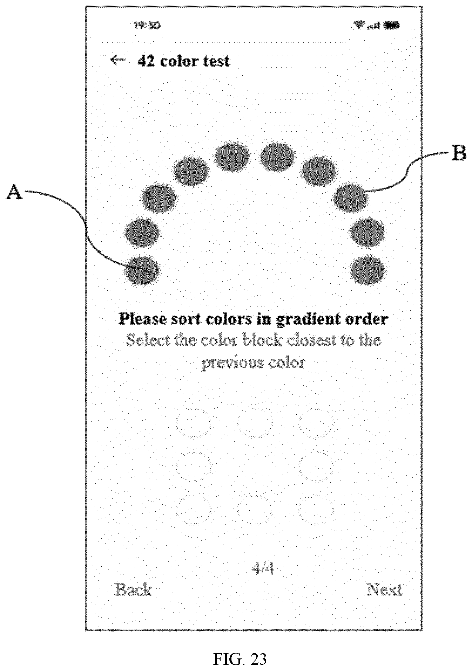

As illustrated in FIG. 17 to FIG. 23, FIG. 17 illustrates a schematic diagram of a tenth kind of interface of the electronic device according to an embodiment of the present disclosure, FIG. 18 illustrates a schematic diagram of an eleventh kind of interface of the electronic device according to an embodiment of the present disclosure, FIG. 19 illustrates a schematic diagram of a twelfth kind of interface of the electronic device according to an embodiment of the present disclosure, FIG. 20 illustrates a schematic diagram of a thirteenth kind of interface of the electronic device according to an embodiment of the present disclosure, FIG. 21 illustrates a schematic diagram of a fourteenth kind of interface of the electronic device according to an embodiment of the present disclosure, FIG. 22 illustrates a schematic diagram of a fifteenth kind of interface of the electronic device according to an embodiment of the present disclosure, and FIG. 23 illustrates a schematic diagram of a sixteenth kind of interface of the electronic device according to an embodiment of the present disclosure.

As illustrated in FIG. 17 to FIG. 23, taking the target number of color blocks as 42 color blocks and the target number of controls to be filled as 42 controls to be filled as examples, 42 color blocks A are divided into 4 groups and 42 controls to be filled are divided into 4 groups, 4 groups of color blocks are displayed in 4 consecutive interfaces corresponding to the first region, and 4 groups of controls to be filled are displayed in 4 consecutive interfaces corresponding to the second region. Specifically, FIG. 17 to FIG. 19 each are the first interface of the four consecutive interfaces, FIG. 20 to FIG. 21 each are the second interface of the four consecutive interfaces, FIG. 22 is the third interface of the four consecutive interfaces, and FIG. 23 is the fourth interface of the four consecutive interfaces.

As illustrated in FIG. 17, in some embodiments, the electronic device may display a part of color blocks A of the 42 color blocks A on the first interface, and display a part of controls to be filled B of the 42 controls to be filled B in the second region, and may highlight the current controls to be filled B2.

As illustrated in FIG. 18, when a drag operation acting on the first color block A3 on the control to be filled B3 is detected, the first color block A3 may be enlarged and displayed.

As illustrated in FIG. 19, when it is detected that a drag operation acting on the first color block A3 on the control to be filled B3 is ended, the normal display of the first color block A3 may be restored.

As illustrated in FIG. 20 and FIG. 21, in some embodiments, the electronic device may display a part of color blocks A of the 42 color blocks A on the second interface, and display a part of controls to be filled B of the 42 controls to be filled B in the second region, and may highlight the current controls to be filled B2.

As illustrated in FIG. 22, in some embodiments, the electronic device may display a part of color blocks A of the 42 color blocks A on the third interface, and display a part of controls to be filled B of 42 controls to be filled B in the second region.

As illustrated in FIG. 23, in some embodiments, the electronic device may display a part of color blocks A of 42 color blocks A on the third interface, and display a part of controls to be filled B of 42 controls to be filled B in the second region.

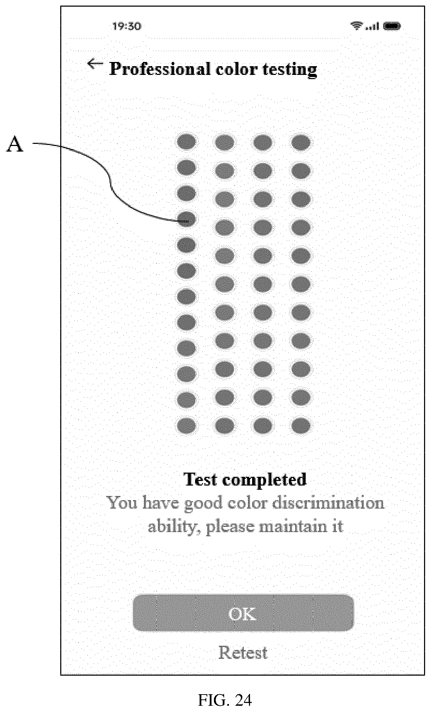

FIG. 24 illustrates a schematic diagram of a seventeenth kind of interface of the electronic device according to an embodiment of the present disclosure. As illustrated in FIG. 24, taking the target number of color blocks as 42 color blocks and the target number of controls to be filled as 42 controls to be filled as examples, after all four groups of tests are completed, when the arrangement orders are consistent with the preset arrangement orders, the target number of color blocks A may be displayed normally after obtaining the test result.

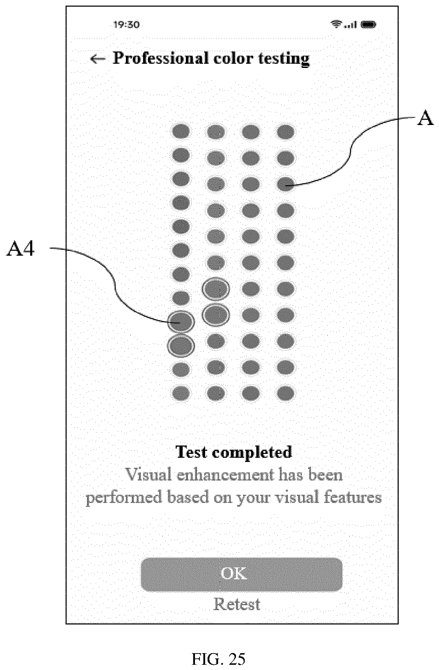

FIG. 25 illustrates a schematic diagram of an eighteenth kind of interface of the electronic device according to an embodiment of the present disclosure. As illustrated in FIG. 25, taking the target number of color blocks as 42 color blocks and the target number of controls to be filled as 42 controls to be filled as examples, after all four groups of tests are completed, when the arrangement orders are inconsistent with the preset arrangement orders, the target number of color blocks A may be displayed and the second color blocks A4 of the target number of color blocks A may be highlighted after obtaining the test result.

At the block S540: filling, in response to a selection operation acting on one of the target number of color blocks, the one of the target number of color blocks to one of the controls to be filled.

At the block S550: in response to detecting that the target number of color blocks are all filled to the target number of controls to be filled, adjusting, based on a relationship between arrangement orders of the target number of color blocks on the target number of controls to be filled and preset arrangement orders, display colors of the electronic device; colors of adjacent color blocks of the target number of color blocks with the preset arrangement orders are most similar.

In some embodiments, the specific description of the blocks S540 to S550 may be found in the blocks S 120 to S 130, which will not be repeated herein.

The method for adjusting display colors according to the further still another embodiment of the present disclosure displays the color testing selection interface. The color testing selection interface includes the first testing selection control and the second testing selection control, and the target number of color blocks corresponding to the testing mode to which the first testing selection control belongs is less than the target number of color blocks corresponding to the testing mode to which the second test control belongs. When the touch operation acting on the first testing selection control is detected, the first color testing interface is displayed. When the touch operation acting on the second testing selection control is detected, the second color testing interface is displayed. In response to the selection operation acting on the color block, the color block is filled to the control to be filled. When it is detected that the target number of color blocks are all filled to the target number of controls to be filled, the display colors of the electronic device are adjusted based on the relationship between the arrangement orders of the target number of color blocks on the target number of controls to be filled and the preset arrangement orders. The colors of the adjacent color blocks of the target number of color blocks in the preset arrangement orders are most similar. Compared to the method for adjusting display colors illustrated in FIG. 1, this embodiment also provides different color block testing modes for the user to choose for testing, so as to cover a wider range of people and enhance the scope of application of display color adjustment.

FIG. 26 illustrates a schematic module block diagram of an apparatus for adjusting display colors according to an embodiment of the present disclosure. The apparatus for adjusting display colors 200 is applied to the electronic device mentioned above. The block diagram illustrated in FIG. 26 will be described below. The apparatus for adjusting display colors 200 includes a color testing interface display module 210, a color block moving module 220, and a display color adjustment module 230.

The color testing interface display module 210 is used to display a color testing interface. The color testing interface includes a first region and a second region, the first region displays a target number of color blocks, and the second region displays a target number of controls to be filled. Each color block is used to fill one of the target number of controls to be filled.

In some embodiments, the color testing interface display module 210 includes: a color testing selection interface display submodule, a first color testing interface display submodule, and a second color testing interface display submodule.

The color testing selection interface display submodule is used to display a color testing selection interface. The color testing selection interface includes a first testing selection control and a second testing selection control. The target number of color blocks corresponding to a testing mode to which the first testing selection control belongs is less than the target number of color blocks corresponding to a testing mode to which the second testing selection control belongs.

The first color testing interface display submodule is used to display a first color testing interface when a touch operation acting on the first testing selection control is detected.

The second color testing interface display submodule is used to display a second color testing interface when a touch operation acting on the second testing selection control is detected.

The color block moving module 220 is used to fill one of the target number of color blocks to the control to be filled in response to a selection operation acting on the one of the target number of color blocks.

The display color adjustment module 230 is used to adjust display colors of the electronic device based on a relationship between arrangement orders of the target number of color blocks on the target number of controls to be filled and preset arrangement orders when it is detected that the target number of color blocks are all filled to the target number of controls to be filled. The colors of adjacent color blocks of the target number of color blocks with the preset arrangement orders are most similar.

In some embodiments, the display color adjustment module 230 includes: a first display color adjustment submodule and a second display color adjustment submodule.

The first display color adjustment submodule is used to acquire attribute information of a user corresponding to the electronic device when the arrangement orders are consistent with the preset arrangement orders, and adjust the display colors of the electronic device based on the attribute information.

The second display color adjustment submodule is used to determine color blocks from the target number of color blocks whose arrangement orders are inconsistent with the preset arrangement orders as second color blocks when the arrangement orders are inconsistent with the preset arrangement orders, and adjust the display colors of the electronic device based on the second color blocks.

In some embodiments, the display color adjustment module 230 includes: a highlighting display submodule.

The highlighting display submodule is used to display the target number of color blocks and highlight the second color blocks.

In some embodiments, the apparatus for adjusting display colors 200 includes: a display cancellation module.

The display cancellation module is used to remove the filling of the first color block on the first control to be filled and keep the first control to be filled in a filling vacancy state when a cancellation operation acting on the first control to be filled is detected.

In some embodiments, the display cancellation module includes a color block moving submodule.

The color block moving submodule is used to move the first color block from the first control to be filled to the first region when the cancellation operation acting on the first control to be filled is detected, or to move the first color block from the first control to be filled to a second control to be filled, where the second control to be filled is any other control to be filled except for the first control to be filled in the target number of controls to be filled.

In some embodiments, the color block moving submodule includes a first color block moving unit.

The first color block moving unit is used to move the first color block from the first control to be filled to the first region when a click operation acting on the first color block on the first control to be filled is detected.

In some embodiments, the color block moving submodule includes: a drag position acquisition unit, a second color block moving unit, and a third color block moving unit.

The drag position acquisition unit is used to obtain a drag position corresponding to the drag operation when a drag operation acting on the first color block on the control to be filled is detected.

The second color block moving unit is used to move the first color block from the first control to be filled to the first region when the drag position exceeds a preset range.

The third color block moving unit is used to move the first color block from the first control to be filled to the second to fill control when the drag position does not exceed the preset range.

In some embodiments, the third color block moving unit includes: a control to be filled determining subunit and a third color block moving subunit.

The control to be filled determining subunit is used to determine a control to be filled closest to the drag position from the target number of controls to be filled as the second control to be filled when the drag position does not exceed the preset range.

The third color block moving subunit is used to move the first color block from the first control to be filled to the second control to be filled.

In some embodiments, the color block moving submodule includes: a highlighting display unit and a restoration display unit.

The highlighting display unit is used to highlight the first color block when the drag operation acting on the first color block on the first control to be filled is detected.

The restoration display unit is used to restore a normal display of the first color block when it is detected that the drag operation acting on the first color block on the first control to be filled is ended.

In some embodiments, the apparatus for adjusting display colors 200 includes a current control to be filled determining module and a highlighting display module.

The current control to be filled determining module is used to determine a current control to be filled from the target number of controls to be filled during a process of filling the target number of color blocks to the target number of controls to be filled.

The highlighting display module is used to highlight the current control to be filled.

Those skilled in the art may clearly understand that, for the convenience and conciseness of description, the specific working processes of the apparatus and modules described above may refer to the corresponding processes in the foregoing method embodiments, which will not be described herein.

In the several embodiments provided by the present disclosure, the modules may be electrically, mechanically, or otherwise coupled to one another.

In addition, in various embodiments of the present disclosure, each functional module may be integrated into one processing module, or each module may physically exist separately, or two or more modules may be integrated into one module. The integrated modules mentioned above may be implemented in the form of hardware or software functional modules.

FIG. 27 illustrates a schematic block diagram of an electronic device 100 according to an embodiment of the present disclosure. The electronic device 100 may be an electronic device capable of running an application program, such as a smart phone, a tablet computer, or an electronic book. The electronic device 100 in the present disclosure may include one or more of the following components: a processor 110, a memory 120, a touch screen 130, and one or more application programs. The one or more application programs may be stored in memory 120 and configured to be executed by one or more processors 110, and the one or more programs may be configured to execute the method described in the aforementioned method embodiments.

In some embodiments, the processor 110 may include one or more processing cores. The processor 110 uses various interfaces and wiring to connect various parts within the entire electronic device 100 to perform various functions and process data of the electronic device 100 by running or executing instructions, programs, code sets, or instruction sets stored in the memory 120, and by calling data stored in the memory 120. Alternatively, the processor 110 may be implemented in at least one hardware form of digital signal processing (DSP), field-programmable gate array (FPGA), or programmable logic array (PLA). The processor 110 may be integrated with one or a combination of a central processing unit (CPU), a graphics processing unit (GPU), and a modem. The CPU is mainly used to process operating systems, user interfaces, and application programs, the GPU is used to render and draw the content to be displayed, and the modem is used to process wireless communication. It can be understood that the above-mentioned modem may also be implemented separately through a communication chip without being integrated into the processor 110.

The memory 120 may include a random access memory (RAM) or a read-only memory (ROM). The memory 120 may be used to store instructions, programs, codes, code sets, or instruction sets. The memory 120 may include a storage program region and a storage data region, the storage program region may store instructions for implementing an operating system, instructions for implementing at least one function (such as touch function, a sound playback function, an image playback function, etc.), instructions for implementing various method embodiments, etc. The storage data region may also store data (such as a phonebook, audio and video data, chat record data) created by the electronic device 100 during use, etc.

The touch screen 130 is used to display information input by the user, information provided to the user, and various graphical user interfaces of the electronic device 100. The graphical user interfaces may be composed of graphics, text, icons, numbers, videos, and any combination thereof. In one embodiment, the touch screen 130 may be a liquid crystal display (LCD) or an organic light emitting diode (OLED), which is not limited in herein.

FIG. 28 illustrates a structural block diagram of a computer-readable storage medium according to an embodiment of the present disclosure. The computer-readable storage medium 300 is stored with program codes, and the program codes are configured to, when being called by a processor, implement the methods described in the above method embodiments.

The computer-readable storage medium 300 may be an electronic memory such as a flash memory, an electrically erasable programmable read-only memory (EEPROM), an erasable programmable read-only memory (EPROM), a hard disk, or a ROM. Alternatively, the computer-readable storage medium 300 includes a non-transitory computer-readable storage medium. The computer-readable storage medium 300 has a storage space for program codes 310 to implement any of steps in the method described above. The program codes may be read from or written into one or more computer program products. The program code 310 may, for example, be compressed in a suitable form.

In summary, the embodiments of the present disclosure provide the method, the apparatus, the electronic device, and the storage medium for adjusting the display colors, in which the color testing interface is displayed. The color testing interface includes the first region and the second region, the first region displays the target number of color blocks, the second region displays the target number of controls to be filled, and each color block is used to fill one control to be filled. In response to the selection operation acting on the color block, the color block is filled into the control to be filled. When it is detected that the target number of color blocks are all filled into the target number of controls to be filled, the display colors of the electronic device are adjusted based on the relationship between the arrangement orders of the target number of color blocks on the target number of controls to be filled and the preset arrangement orders. The colors of adjacent color blocks of the target number of color blocks with the preset arrangement orders are most similar. Therefore, by providing the color testing interface for sorting similar colors and adjusting the display colors of the electronic device based on the test result, personalized display colors can be achieved and the user experience can be improved.

Finally, it should be noted that the above embodiments are merely used to illustrate the technical solutions of the present disclosure, not to limit them. Although the present disclosure has been described in detail with reference to the aforementioned embodiments, those skilled in the art should understand that the technical solutions described in the foregoing embodiments may still be modified, or some of the technical features thereof may be equivalently replaced. These modifications or replacements do not drive the essence of the corresponding technical solutions deviate from the spirit and scope of the technical solutions of the various embodiments of the present disclosure.

## Claims

1. A method for adjusting display colors, implemented by an electronic device, the method comprising:
displaying a color testing interface, wherein the color testing interface comprises a first region and a second region, the first region displays a target number of color blocks, the second region displays a target number of controls to be filled, and each of the target number of color blocks is configured to fill one of the target number of controls to be filled;
filling, in response to a selection operation acting on one of the target number of color blocks, the one of the target number of color blocks to one of the controls to be filled; and
in response to detecting that the target number of color blocks are all filled to the target number of controls to be filled, adjusting, based on a relationship between arrangement orders of the target number of color blocks on the target number of controls to be filled and preset arrangement orders, display colors of the electronic device; wherein colors of adjacent color blocks of the target number of color blocks with the preset arrangement orders are most similar.

2. The method according to claim 1, wherein the target number of color blocks comprise a first color block, the target number of controls to be filled comprise a first control to be filled, the first control to be filled is filled with the first color block, and after the filling, in response to a selection operation acting on one of the target number of color blocks, the one of the target number of color blocks to one of the controls to be filled, the method comprises:
removing, in response to detecting a cancellation operation acting on the first color block on the first control to be filled, the filling of the first color block on the first control to be filled and keeping the first control to be filled in a filling vacancy state.

3. The method according to claim 2, wherein the removing, in response to detecting a cancellation operation acting on the first color block on the first control to be filled, the filling of the first color block to the first control to be filled and keeping the first control to be filled in a filling vacancy state comprises:
in response to detecting the cancellation operation acting on the first color block on the first control to be filled, moving the first color block from the first control to be filled to the first region, or moving the first color block from the first control to be filled to a second control to be filled, wherein the second control to be filled is any other control to be filled in the target number of controls to be filled except for the first control to be filled.

4. The method according to claim 3, wherein the moving the first color block from the first control to be filled to the first region, in response to detecting the cancellation operation acting on the first color block on the first control to be filled, comprises:
moving, in response to detecting a click operation acting on the first color block on the first control to be filled, the first color block from the first control to be filled to the first region.

5. The method according to claim 4, wherein the moving, in response to detecting a click operation acting on the first color block on the first control to be filled, the first color block from the first control to be filled to the first region comprises:
moving, in response to detecting the click operation acting on the first color block on the first control to be filled, the first color block from the first control to be filled to an initial display position in the first region; or
moving, in response to detecting the click operation acting on the first color block on the first control to be filled, the first color block from the first control to be filled to any vacant position in the first region.

6. The method according to claim 3, wherein the moving the first color block from the first control to be filled to the first region or moving the first color block from the first control to be filled to a second control to be filled, in response to detecting the cancellation operation acting on the first color block on the first control to be filled, comprises:
obtaining, in response to detecting a drag operation acting on the first color block on the first control to be filled, a drag position corresponding to the drag operation;
moving, in response to the drag position exceeding a preset range, the first color block from the first control to be filled to the first region;
moving, in response to the drag position not exceeding the preset range, the first color block from the first control to be filled to the second control to be filled.

7. The method according to claim 6, wherein the moving, in response to the drag position not exceeding the preset range, the first color block from the first control to be filled to the second control to be filled comprises:
determining, in response to the drag position not exceeding the preset range, a control to be filled closest to the drag position from the target number of controls to be filled as the second control to be filled; and
moving the first color block from the first control to be filled to the second control to be filled.

8. The method according to claim 6, further comprising:
highlighting, in response to detecting the drag operation acting on the first color block on the first control to be filled, the first color block; and
restoring, in response to detecting that the drag operation acting on the first color block on the first control to be filled is ended, a normal display of the first color block.

9. The method according to any one of claims 1-8, wherein the adjusting, based on a relationship between arrangement orders of the target number of color blocks on the target number of controls to be filled and preset arrangement orders, display colors of the electronic device comprises:
acquiring, in response to the arrangement orders being consistent with the preset arrangement orders, attribute information of a user corresponding to the electronic device and adjusting the display colors of the electronic device based on the attribute information; or
determining, in response to the arrangement orders being inconsistent with the preset arrangement orders, color blocks whose arrangement orders are inconsistent with the preset arrangement orders from the target number of color blocks as second color blocks and adjusting the display colors of the electronic device based on the second color blocks.

10. The method according to claim 9, wherein the determining, in response to the arrangement orders being inconsistent with the preset arrangement orders, color blocks whose arrangement orders are inconsistent with the preset arrangement orders from the target number of color blocks as second color blocks and adjusting the display colors of the electronic device based on the second color blocks comprises:
determining, in response to the arrangement orders being inconsistent with the preset arrangement orders, the color blocks whose arrangement orders are inconsistent with the preset arrangement orders from the target number of color blocks as the second color blocks and obtaining the number of the second color blocks;
performing weight analysis on the second color blocks and the number of the second color blocks to obtain an analysis result; and
adjusting, based on the analysis result, the display colors of the electronic device.

11. The method according to claim 9, further comprising:
displaying the target number of color blocks and highlighting the second color blocks.

12. The method according to any one of claims 1-11, wherein the electronic device comprises a touch screen, and the displaying a color testing interface comprises:
calibrating, in response to an instruction to enter the color testing interface, the touch screen to a uniform color; and
displaying the color testing interface on the calibrated touch screen.

13. The method according to any one of claims 1-11, wherein the displaying a color testing interface comprises:
displaying a color testing selection interface, the color testing selection interface comprising a first testing selection control and a second testing selection control, wherein the target number of color blocks corresponding to a testing mode to which the first testing selection control belongs is less than the target number of color blocks corresponding to a testing mode to which the second testing selection control belongs;
displaying, in response to detecting a touch operation acting on the first testing selection control, a first color testing interface;
displaying, in response to detecting a touch operation acting on the second testing selection control, a second color testing interface.

14. The method according to claim 13, wherein when the first color testing interface is displayed, the target number of color blocks are displayed through a current interface corresponding to the first region, and when the second color testing interface is displayed, the target number of color blocks are displayed through a plurality of consecutive interfaces corresponding to the first region.

15. The method according to claim 14, wherein the testing mode to which the first testing selection control belongs corresponds to 16 color blocks, and the testing mode to which the second testing selection control belongs corresponds to 42 color blocks.

16. The method according to any one of claims 1-15, further comprising:
determining a current control to be filled from the target number of controls to be filled during a process of filling the target number of color blocks into the target number of controls to be filled; and
highlighting the current control to be filled.

17. The method according to any one of claims 1-16, wherein after the displaying a color testing interface, the method further comprises:
exiting, in response to detecting a cancellation testing operation acting on the color testing interface, the color testing interface.

18. An apparatus for adjusting display colors, applied to an electronic device, comprising:
a color testing interface display module, configured to display a color testing interface, wherein the color testing interface comprises a first region and a second region, the first region displays a target number of color blocks, the second region displays a target number of controls to be filled, and each of the target number of color blocks is configured to fill one of the target number of controls to be filled;
a color block moving module, configured to fill, in response to a selection operation acting on one of the target number of color blocks, the one of the target number of color blocks to one of the controls to be filled; and
a display color adjustment module, configured to: in response to detecting that the target number of color blocks are all filled to the target number of controls to be filled, adjust, based on a relationship between arrangement orders of the target number of color blocks on the target number of controls to be filled and preset arrangement orders, display colors of the electronic device; wherein colors of adj acent color blocks of the target number of color blocks with the preset arrangement orders are most similar.

19. An electronic device, comprising a memory and a processor, wherein the memory is coupled to the processor, the memory is stored with instructions which, when being executed by the processor, cause the processor to implement the method according to any one of claims 1-17.

20. A computer readable storage medium, wherein the computer readable storage medium is stored with program codes, and the program codes are capable of being called by a processor to implement the method according to any one of claims 1-17.
